# DEMANDE DE BREVET EUROPEEN

(11) **EP 1 508 328 A1**
(43) Date de publication de la demande: **23.02.2005**
(21) Numéro de dépôt: 04291941.5
(22) Date de dépôt: 29.07.2004
(51) Int. Cl.: A61K 7/48, A61K 31/00, A61K 31/385, A61K 31/165, A61K 31/19, A61K 45/06

(54) **Composition destinée à lutter contre la dégradation des fibres de collagène induite en condition d'exposition solaire naturelle**

(30) Priorité: 22.08.2003 FR 0310103
(71) Demandeur: L'OREAL, 75008 Paris (FR)
(72) Inventeur: Fagot, Dominique, 75020 Paris (FR); Bernerd, Francoise, 75018 Paris (FR)
(74) Mandataire: Allab, Myriam

(57) **Abrégé**

L'invention concerne l'utilisation, pour la préparation d'une composition cosmétique ou dermatologique destinée à prévenir et/ou lutter contre la dégradation des fibres de collagène induite en condition d'exposition solaire naturelle, en particulier lorsque le rapport UVA/UVB est compris entre 10 et 17, d'une quantité efficace d'au moins un inhibiteur de la production de facteurs cytosolubles kératinocytaires photoinduits. En particulier, l'inhibiteur est le butyrate de sodium.

L'invention porte également sur des compositions cosmétiques particulières ainsi que sur un procédé cosmétique de traitement de la peau et/ou du cuir chevelu.

## Description

L'invention concerne l'utilisation, pour la préparation d'une composition cosmétique ou dermatologique destinée à prévenir et/ou lutter contre la dégradation des fibres de collagène induite par un rayonnement solaire caractérisé par un rapport UVA/UVB compris entre 10 et 17, d'une quantité efficace d'au moins un inhibiteur de la production de facteurs cytosolubles kératinocytaires photoinduits.
Elle a également pour objet l'utilisation, pour la préparation d'une composition cosmétique ou dermatologique destinée à prévenir et/ou lutter contre la dégradation photoinduite des fibres de collagène, d'inhibiteurs de facteurs cytosolubles kératinocytaires spécifiques.
Les compositions comprenant lesdits inhibiteurs sont destinées à inhiber la production de MMP-1 au niveau du derme, induite en présence d'une exposition solaire naturelle sub-érythémale ou érythémale.

L'invention a également pour objet des compositions comprenant des inhibiteurs particuliers et un procédé cosmétique destiné à prévenir et/ou diminuer les signes cutanés du vieillissement et/ou destiné à freiner la chute des cheveux.

Par 'exposition solaire naturelle' selon l'invention, on entend notamment une exposition solaire couvrant les variations réelles de rayonnement solaire en terme de dose et de rapport UVA/UVB, notamment la condition zénithale, lesdites variations pouvant correspondre à une exposition solaire qualifiée d'érythémale ou à une exposition solaire qualifiée de sub-érythémale, selon qu'elle déclenche ou non un érythème inflammatoire.

Par inhibiteurs 'de la production' de facteurs cytosolubles kératinocytaires selon l'invention, on entend notamment tout agent capable d'inhiber (i) les voies de signalisation conduisant à la transcription, la traduction, (ii) les processus de modification post-traductionelle, (iii) la sécrétion et/ou (iv) l'activation intra- et/ou extra-cellulaire de facteurs cytosolubles kératinocytaires.
La même définition est applicable aux inhibiteurs 'de la production' des collagénases.

La peau humaine est constituée de deux compartiments à savoir un compartiment superficiel, l'épiderme, et un compartiment profond, le derme.
L'épiderme humain naturel est composé principalement de trois types de cellules qui sont les kératinocytes, très majoritaires, les mélanocytes et les cellules de Langerhans. Chacun de ces types cellulaires contribue par s es fonctions propres au rôle essentiel joué dans l'organisme par la peau.
Le derme fournit à l'épiderme un support solide. C'est également son élément nourricier. II est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également traversé par des vaisseaux sanguins et des fibres nerveuses.

Ce sont les fibres de collagène qui assurent la solidité du derme. Les fibres de collagène sont constituées de fibrilles scellées les unes aux autres, formant ainsi plus de dix types de structures différentes. La solidité du derme est en grande partie due à l'enchevêtrement de bonne qualité des fibres de collagène tassées les unes contre les autres en tous sens. Les fibres de collagène participent à la fermeté, l'élasticité et la tonicité de la peau et/ou des muqueuses.
Elles sont constamment renouvelées mais ce renouvellement diminue avec l'âge ce qui entraîne un amincissement du derme.

En outre, divers facteurs, dont les rayonnements ultraviolets, entraînent la dégradation du collagène, avec toutes les conséquences q ue l'on peut envisager sur la structure et/ou la fermeté de la peau et/ou des muqueuses, en particulier sur les zones du corps habituellement exposées au soleil : le visage, les oreilles, le cuir chevelu, le cou, les avant-bras et les mains.

Les dommages cutanés liés à une exposition chronique (irradiation répétée) ou aiguë (irradiation forte) aux UV-A ou aux UV-B ont été bien étudiés ; on sait notamment que :
- les UV-B (290-300nm ; 5% des UV totaux), de longueurs d'ondes les plus énergétiques, affectent surtout les cellules épidermiques (kératinocytes), en agissant au niveau de l'ADN ;
- les UV-A (320-400nm ; 95% des UV totaux), plus pénétrantes, atteignent les cellules dermiques telles que les fibroblastes et agissent indirectement via la génération de radicaux libres ;
- de plus, une exposition prolongée aux rayonnements ultraviolets, particulièrement aux rayonnements ultraviolets de type A et/ou B, a pour effet une stimulation de l'expression des collagénases, particulièrement de la métalloprotéinase matricielle de type 1 (MMP-1).

C'est là une des composantes du vieillissement cutané photo-induit. En outre, on sait que l'activité des MMP-1, MMP-2 et MMP-9 augmente avec l'âge et que cette augmentation contribue, avec le ralentissement de la croissance cellulaire, au vieillissement chronologique de la peau (WO 98/36742).

Les fibres de collagène, bien que très résistantes sont cependant sensibles à certaines enzymes.
Ces protéines font partie d'une famille d'enzymes appelées métalloprotéinases matricielles (MMPs) qui sont elles-mêmes les membres d'une famille d'enzymes protéolytiques (endoprotéases) qui possèdent un atome de zinc coordonné à 3 résidus cystéine et une méthionine dans leur site actif et qui peuvent, collectivement, dégrader les composants macromoléculaires de la matrice extracellulaire et des lames basales à pH neutre (collagène, élastine, etc....).

La famille des métalloprotéinases est ainsi constituée de plusieurs groupes bien définis basés sur leurs ressemblances en terme de structure et de spécificité de substrat (voir Woessner J. F., Faseb Journal, vol. 5, 1991, 2145). Parmi ces groupes, on peut citer les collagénases destinées à dégrader les collagènes fibrillaires (MMP-1 ou collagénase interstitielle, MMP-8 ou collagénase neutrophilaire, MMP-13 ou collagénase 3), les gélatinases qui dégradent le collagène de type IV ou toute forme de collagène dénaturé (MMP-2 ou gélatinase A (72 kDa), MMP-9 ou gélatinase B (92 kDa), les stromélysines (MMP-3 ou stromélysine 1, MMP-10 ou stromélysine 2, MMP-11 ou stromélysine 3) dont le large spectre d'activité s'adresse aux protéines de la matrice extracellulaire telles que les glycoprotéines (fibronectine, laminine), les protéoglycannes, etc., la matrilysine (MMP-7), la métalloélastase (MMP-12) ou encore les métalloprotéinases membranaires (MMP-14, MMP-15, MMP-16 et MMP-17).
Les métalloprotéinases sont produites et sécrétées sous une forme inactive (pro-enzyme). Ces formes inactives dites zymogènes sont ensuite activées dans l'environnement extracellulaire par l'élimination d'une région propeptidique. Les membres de cette famille peuvent s'activer les uns les autres. La régulation de l'activité des MMPs se produit ainsi au niveau de l'expression des gènes (transcription et traduction), au niveau de l'activation de la forme zymogène, ou au niveau du contrôle local des formes actives.

On comprend donc l'importance du collagène dans la structure des tissus, particulièrement de la peau et/ou des muqueuses, et l'importance qu'il y a à prévenir et/ou combattre sa dégradation pour ainsi lutter contre le vieillissement qu'il soit chronobiologique ou photo-induit et ses conséquences, l'amincissement du derme et/ou l'apparence de la peau molle et/ou ridée.
On sait par ailleurs que les MMPs sont impliquées dans la dégradation de la matrice périfolliaire et donc la chute des cheveux.

On pensait jusqu'ici que le processus de photovieillissement cutané et en particulier l'induction de métalloprotéinases responsables de la dégradation des fibres de collagène au niveau du derme, était mis en oeuvre lorsque la peau était soumise à un rayonnement UV capable d'induire un érythème solaire (rougeur inflammatoire, coup de soleil), et défini par une dose minimale érythémale (DME ou MED). Cette dose varie en fonction du phototype de l'individu et du rapport UVA/UVB.

Les études antérieures ont donc cherché à reproduire des conditions d'exposition solaire comprenant des rayonnements UVA et/ou UVB, à des doses autour de la MED, pour identifier des composés capables de protéger la peau contre les effets néfastes du soleil, et en particulier contre l'érythème, associé indirectement au photovieillissement. Mais à la connaissance de la Demanderesse, aucune des études réalisées jusqu'ici n'a permis de reproduire des conditions proches d'une exposition solaire naturelle. En effet, dans les études de l'art antérieur :
- soit le rayonnement UV est appliqué à des doses qualifiées de fortes (par exemple 2MED, Laman et al., 2001, Photochem. Photobiol., 73, 657-663) ;
- soit le rayonnement U V est caractérisé par un rapport UVA/UVB déséquilibré, très en faveur des UVB, responsables de l'érythème (100%UVB, Fagot et al., 2002, Arch Dermatol Res, 293 : 576-583).
   Il existe donc un besoin d'identifier de nouveaux composés qui soient efficaces, non pas seulement vis-à-vis de l'érythème, mais également dans des conditions d'exposition solaire naturelle, c'est-à-dire vis-à-vis d'événements plus précoces et moins perceptibles liés aux effets néfastes du soleil, comme une altération progressive des tissus cutanés responsable, avec le temps et la répétition des expositions solaires, des signes cutanés du photo-viellissement (rides, ridules, peau molle, amincie...). Ces événements précoces selon l'invention comprennent notamment la dégradation de la matrice extracellulaire et des protéines comme le collagène et l'élastine, en particulier la dégradation des fibres de collagène induite par l'action des métalloprotéinases et en particulier de MMP1.

Lahman et al. ont décrit, en condition de radiation solaire simulée RSS et à une dose égale à 2 fois la dose minimale érythémale (2MED), donc simulant des conditions d'exposition solaire érythémale, une production de MMP-1 au niveau du derme (fibroblastes) et de l'épiderme (kératinocytes).

De manière inattendue, la Demanderesse vient de démontrer que dans des conditions proches d'une exposition solaire naturelle sub-érythémale, c'est-à-dire en condition de radiation solaire simulée (RSS) à une dose sub-érythémale, et avec un rapport UVA/UVB compris entre 10 et 17, les kératinocytes ne sont pas capables de produire des MMP-1, mais participent néanmoins à la production de MMP-1 par les fibroblastes du derme, *via* un mécanisme paracrine impliquant des facteurs cytosolubles kératinocytaires. En outre, la Demanderesse a pu montrer que l'effet stimulateur sur la production de MMP-1 fibroblastique est 18 fois plus important en présence de facteurs cytosolubles kératinocytaires photoinduits que par une irradiation directe des fibroblastes (facteur 8).

C'est donc la première fois que l'on met en évidence, dans des conditions et des doses proches de celles d'une exposition solaire naturelle, en particulier en condition sub-érythémale, le rôle respectif de l'épiderme et du derme dans la production de MMP-1 au niveau du derme et la mise en oeuvre d'un mécanisme paracrine impliquant des facteurs cytosolubles kératinocytaires.

Par 'dose sub-érythémale' selon l'invention, on entend une dose biologique efficace (DBE ou BED) capable d'induire la formation de cellules coups de soleil dans l'épiderme, caractérisées notamment par une forme ronde et une perte de connection avec les kératinocytes, ladite dose n'induisant pas un érythème inflammatoire.
En particulier, la dose BED selon l'invention est de 10J/cm² en RSS, soit 9.2J/cm² d'UVA et 0.8J/cm² d'UVB, pour une RSS de 2.34mW/cm².

Par 'radiation solaire simulée' (RSS) selon l'invention, on entend un spectre solaire UV entier simulé, comprenant au moins des UV de type A et de type B (290-400nm).
Cette radiation solaire simulée est généralement obtenue en présence d'un système associant au moins une lampe et des filtres. On utilisera par exemple une lampe au xénon de 1000 watt (Oriel corp. CT) équipée de filtres Schoot UG 5/2mm et WG 320/1.5mm, pour fournir un spectre UV solaire s imulé (290-400nm). G énéralement, la sortie spectrale délivrée correspond à une condition réelle au zénith (8% UVB).

Par 'mécanisme paracrine' selon l'invention, on entend notamment un mécanisme mettant en oeuvre des facteurs cytosolubles produits par des cellules de l'épiderme (kératinocytes) et capables de diffuser jusqu'aux cellules cibles (fibroblastes) pour empêcher la production de MMP1 par lesdites cellules.

Ces résultats ouvrent donc de nouvelles perspectives dans la prévention et/ou le traitement des événements précoces liés aux effets néfastes du soleil, comme la dégradation des fibres de collagène au niveau du derme, induite notamment par un rayonnement solaire naturel caractérisé par un rapport UVA/UVB compris entre 10 et 17. Ce rapport UVA/UVB couvre les variations de quantités de radiation d'UVA et d'UVB, selon que l'on est dans des conditions avec une quantité élevée d'UV-B (soleil au zénith, ensoleillement estival) ou dans des situations à faible quantité d'UV-B et forte quantité d'UV-A (condition non zénithale).
La Demanderesse a également montré que l'utilisation d'une quantité efficace de butyrate de sodium permettait *in vitro* de diminuer cet effet stimulateur sur la production de MMP1, en inhibant la production de facteurs cytosolubles kératinocytaires photoinduits.

On connaît de l'art antérieur l'utilisation thérapeutique du butyrate de sodium comme agent inhibiteur de la prolifération des muscles lisses du système vasculaire (US 5 563 173) ou comme agent anti-cancéreux (Saunders N et al., Cancer research 59, 399-404, January 15, 1999). EP 0 345 081 décrit également des comprimés oraux ou onguents destinés à traiter la chromatose, contenant du butyrate de calcium, un autre sel de l'acide butyrique, en tant qu'agent inhibiteur de la formation de la tyrosinase.

Mais il n'est nullement suggéré l'utilisation d'inhibiteurs d'histones d éacétylases et en particulier de dérivés et/ou sels d'acide butyrique pour la préparation de compositions cosmétiques ou dermatologiques destinées à prévenir la dégradation du collagène induite en condition d'exposition solaire sub-érythémale ou érythémale.

Un premier objet de l'invention est donc l'utilisation, pour la préparation d'une composition cosmétique ou dermatologique destinée à prévenir et/ou lutter contre la dégradation des fibres de collagène induite par un rayonnement solaire caractérisé par un rapport UVA/UVB compris entre 10 et 17, d'une quantité efficace d'au moins un inhibiteur de la production de facteurs cytosolubles kératinocytaires photoinduits. L'inhibiteur d e la p roduction d e facteurs cytosolubles k ératinocytaires p hotoinduits e st utilisé dans la composition comme agent destiné à prévenir et/ou lutter de façon précoce contre la dégradation des fibres de collagène induite en condition d'exposition solaire naturelle sub-érythémale ou érythémale.

Par 'rayonnement solaire' selon l'invention, on entend notamment tout rayonnement équivalent au spectre solaire naturel, en particulier au spectre zénithal, et comprenant au moins des UVA et des UVB dans un rapport UVA/UVB compris entre 10 et 17, de préférence supérieur ou égal à 11 et inférieur ou égal à 16.
En particulier, le rayonnement solaire selon l'invention pourra comprendre 92% d'UVA et 8% d'UVB, reproduisant les conditions du spectre solaire zénithal dans le domaine UVB. De préférence, la proportion d'UVB dans le rayonnement solaire défini selon l'invention sera comprise entre 6 et 9%.

L'utilisation selon l'invention permet ainsi de protéger efficacement la peau tout au long de la journée et ainsi prévenir et/ou lutter de façon précoce contre la dégradation des fibres de collagène, induite par une exposition solaire naturelle sub-érythémale ou érythémale.
Elle présente en outre l'avantage de s'affranchir des problèmes de biodisponibilité des inhibiteurs de MMPs classiques, du fait que les inhibiteurs de facteurs cyosolubles kératinocytaires selon l'invention ont pour cible des cellules épidermiques (kératinocytes) et non des cellules dermiques.
On qualifiera les inhibiteurs de MMPs selon l'invention d'inhibiteurs 'indirects', par opposition aux inhibiteurs connus jusqu'ici, qualifiés d'inhibiteurs 'directs' (action directement au niveau du derme, site de production des MMP1).

L'inhibition de la dégradation photoinduite des fibres de collagène au niveau du derme, en condition d'exposition solaire naturelle sub-érythémale ou érythémale permet notamment d'envisager les applications suivantes :
- prévenir et/ou diminuer et/ou traiter les signes cutanés du photo-vieillissement, en particulier l'amincissement de la peau et/ou la perte de tonicité de la peau et/ou la perte d'élasticité de la peau ;
- et indirectement prévenir et/ou lutter de façon précoce contre les atteintes liées à la ménopause, aggravées par la présence de MMP1 photoinduite ;
- induire et/ou stimuler la croissance des cheveux et/ou freiner leur chute, aggravée par la présence de MMP1 photoinduite.

Par 'signes cutanés du vieillissement', on entend notamment toute modification de l'aspect extérieur de la p eau due au vieillissement chronobiologique et/ou photoinduit, comme les rides et ridules, la peau flétrie, la peau molle, la peau amincie, le manque d'élasticité et/ou de tonus de la peau, ainsi que toute modification interne de la peau, comme toute dégradation interne de la peau, en particulier du collagène, consécutives à une exposition aux rayonnements ultra-violets.
Par 'atteintes cutanées liées à la ménopause', on entend notamment une peau amincie, un manque d'élasticité et une accentuation des rides et des ridules.
En effet, à la ménopause, les principales modifications concernant le derme sont une altération du tissu élastique et une diminution du taux de collagène et de l'épaisseur dermique. Cela entraîne chez la femme ménopausée un amincissement de la peau et/ou des muqueuses. La femme ressent alors une sensation de "peau sèche" ou de peau qui tire et l'on constate une accentuation des fines rides et ridules de surface. La peau présente un aspect rugueux à la palpation. Enfin la peau présente une souplesse diminuée.

L'inhibiteur de la production de facteurs cytosolubles kératinocytaires photoinduits utilisé selon l'invention est susceptible d'être sélectionné selon le procédé comprenant les étapes suivantes :
a- on soumet des kératinocytes humains normaux cultivés en monocouche à une radiation solaire simulée ou un rayonnement UV, caractérisés par un rapport UVA/UVB compris entre 10 et 17 comme décrit précédemment ;
b- on met les cellules kératinocytaires irradiées ou non au contact d'un milieu de culture contenant ou non le produit à tester ;
c- on met le milieu conditionné issu de b) au contact de fibroblastes humains normaux cultivés en monocouche ;
d- on mesure la production de collagénase interstitielle MMP-1 dans le surnageant de culture des fibroblastes issu de c), que l'on compare à un témoin constitué d'une culture de fibroblastes normaux humains mis en contact avec du milieu conditionné de cellules kératinocytaires non irradiées; et
e- on sélectionne les produits pour lesquels la mesure de la production de collagénase interstitielle MMP-1 est inférieure d'au moins 10% à celle obtenue pour le témoin, préférentiellement inférieure d'au moins 20%, et encore plus préférentiellement inférieure d'au moins 30% à celle obtenue pour le témoin.

Par 'milieu conditionné', on entend un milieu de culture mis au contact de cellules puis duquel on a enlevé lesdites cellules. Ce milieu contient donc les facteurs cytosolubles produits par les cellules.

D'autres méthodes susceptibles de mettre en évidence une différence d'inhibition sur la production de MMP-1 par rapport à un témoin peuvent également être utilisées, à condition de mettre en oeuvre les modèles cellulaires et le milieu conditionné décrits. On peut citer par exemple les méthodes connues de mesure de la production d'ARN (PCR, Northern) ou de protéines ou de leurs activités enzymatiques (Western, ELISA, zymogrammes) appliquées à tous modèles cellulaires d'épiderme (épidermes reconstruits de type EPISKIN contenant ou non des cellules de Langherans tels que décrits dans les demandes EP 502 172 et EP 789 074), ou de derme (fibroblastes en monocouche ou insérés dans des lattices de collagène ; ou cellules souches mésenchymateuses de fibroblastes).

L'inhibiteur de la production de facteurs cytosolubles kératinocytaires photoinduits selon l'invention est préférentiellement choisi parmi un inhibiteur de la production de cytokines inflammatoires, un inhibiteur des cyclooxygénases, un inhibiteur des histones déacétylases et leurs mélanges.
En particulier, on utilisera un inhibiteur d'histones déacétylases.

Par 'inhibiteur de cytokines inflammatoires', on entend notamment tout agent capable d'inhiber la production d'interleukines IL1, IL6 et/ou TNFalpha (Tumor Necrosis Factor) ; on peut citer par exemple les activateurs des 'peroxysome proliferator-activated receptors= PPAR), comme le clofibrate ou WY14,643.

Par 'inhibiteur des cyclooxygénases', on entend désigner toute substance permettant, *in vivo,* de limiter ou d'inhiber totalement la transcription, la traduction et/ou l'activité enzymatique d es cyclo-oxygénases. L'activité i nhibitrice vis-à-vis d es cylco-oxygénases peut être mesurée par exemple par des méthodes d'analyses chromatographiques HPLC 5Huang M. et al., cancer Res. 51, pp 813-819, 1991) ou par radioimmunoessais (Lysz T.W. et Needleman P.J, Neurochim., 38, pp 1111-1117, 1982). On peut encore mentionner le test décrit dans Salvameni et al., Proc. Natl. Sci. USA, 90, pp 7240-7244, 1993).

On peut citer par exemple un inhibiteur de la cyclooxygénase-2 comme le Celecoxib.
D'autres inhibiteurs de cyclo-oxygénases peuvent être choisis parmi les agents anti-inflammatoires non-stéroïdiens tels que les dérivés arylcarboxyliques, les dérivés pyrazolés, les dérivés de l'oxicam, les dérivés de l'acide nicotinique.
On peut encore citer l'acide méclofenamique, l'acide méfénamique, le carprofen, le diclofenac, le diflunisal, le fenbufen, le fenoprofen, l'ibuprofen, l'indométhacine, le ketoprofen, la nabumetone, le naproxen, le sulindac, le tenoxicam, la tolmetine, ou encore l'acide acetylsalicylique.
On pourra encore utiliser les corticoïdes tels que la dexaméthasone ou l'hydrocortisone, les fénamates tels que le morniflumate, les dérivés de chalcone tels que le 3,4-dihydroxychalcone, ou les inhibiteurs de phospholipase A₂.

Par 'inhibiteur d'histones déacétylases', on entend notamment l'acide α-lipoïque, la trichostatin A, et un acide gras à chaîne courte en C2 à C6 tel que l'acide propionique et/ou ses dérivés et/ou ses sels, l'acide butyrique et/ou des dérivés et/ou ses sels, et leurs mélanges.
Selon un mode particulier de l'invention, on utilisera tout inhibiteur d'histones déacétylases à l'exception de l'acide α-lipoïque.

Parmi les 'dérivés', on peut citer notamment les esters, et en particulier ceux comprenant des radicaux sucres et hydroxydes. On peut citer par exemple le monoacétone glucose-3-propionate et le monoacétone glucose-3-butyrate.
Par 'sels' selon l'invention, on entend notamment les sels organiques ou inorganiques. Parmi les sels organiques, on peut citer les sels ammoniums obtenus par réaction avec des amino-acides basiques.
Parmi les sels inorganiques, on peut citer notamment les sels obtenus par réaction avec les métaux alcalins (sodique ou potassique), les alcalino-terreux (Mg2+, Ca2+) et les métaux de transition (Fe, Mn, Co, Cu...). On pourra utiliser par exemple le propionate de sodium ou le butyrate de sodium, et préférentiellement le butyrate de sodium.

Comme dérivés de l'acide butyrique utilisables selon l'invention, on peut citer les composés de formule générale (I): 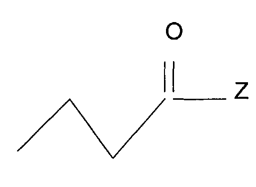 où Z peut être un groupe choisi parmi les groupes -OH, -OR', -NH2, -NHR', -NR'R" ; et R' et R" peuvent être des groupes aryle ou alkyle en C1-C8, linéaires ou ramifiés, saturés ou insaturés, éventuellement substitués par des groupes hydroxyles, alkoxy, acyloxy, amino, alkylamino, ou aryles, et/ou leurs sels.

Comme sels organiques d'acide butyrique ou de ses dérivés de formule (I) utilisables selon l'invention, on peut citer les sels de tri-éthanolamine, mono-éthanolamine, di-éthanolamine, hexadécylamine et N,N,N',N'-tétrakis-(hydroxy-propyl-2) éthylène diamine, tris-hydroxyméthyl-aminométhane ou encore les sels d'acides carboxyliques comme les citrates, les acétates, les lactates, les fumarates, les gluconates, les oxalates pour les composés selon l'invention portant une fonction basique.

Comme sels inorganiques d'acide butyrique ou de ses dérivés de formule (I) utilisables selon l'invention, on peut citer les sels d'acides minéraux comme les sels de sodium ou de potassium, les sels de zinc (Zn²⁺), de calcium (Ca²⁺), de cuivre (Cu²⁺), de fer (Fe²⁺, Fe³⁺), de strontium (Sr²⁺), de magnésium (Mg²⁺) et de manganèse (Mn²⁺) ; ou encore les hydroxydes, les carbonates ou hydrogénocarbonates, les chlorures, les sulfates, les phosphates ou hydrogénophosphates pour les compososés selon l'invention portant une fonction basique.

De préférence, on utilisera le butyrate de sodium.

Parmi les facteurs cytosolubles kératinocytaires induits en réponse à une irradiation UV comme cibles de l'inhibiteur objet de la présente invention, on peut notamment citer ceux décrits dans Sesto A. et al., Proc Natl Acad Sci USA 2002 Mar 5 ;99(5) :2965-70 et Li D. et al., FASEB J. 2001 Nov ; 15(13) :2533-5.

Un autre objet de l'invention est l'utilisation, pour la préparation d'une composition cosmétique ou dermatologique destinée à prévenir et/ou lutter contre la dégradation photoinduite des fibres de collagène, d'une quantité efficace d'au moins un inhibiteur de facteurs cytosolubles kératinocytaires photoinduits choisis parmi un inhibiteur d'histones déacétylases, un inhibiteur de cyclooxygénases et leurs mélanges.La composition et/ou l'inhibiteur seront utiles en tant qu'inhibiteur de la production de métalloprotéinase (MMPs), en particulier inhibiteur de la collagénase MMP-1 au niveau du derme. Par 'inhiber' la production de MMPs, on entend diminuer voire inhiber la production de MMPs.

De préférence, on utilisera comme inhibiteur de facteurs cytosolubles kératinocytaires, un inhibiteur d'histone déacétylases choisi parmi l'acide α-lipoïque, la trichostatin A, un acide gras à chaîne courte en C2 à C6 tel que l'acide propionique et/ou ses dérivés et/ou ses sels et l'acide butyrique et/ou ses dérivés et/ou ses sels, et leurs mélanges, tels q ue définis précédemment.
Selon un mode préféré, on utilisera le butyrate de sodium ou le monoacétone 3-glucose butyrate.

L'inhibiteur de facteurs cytosolubles kératinocytaires photoinduits selon l'invention sera généralement utilisé en une quantité comprise entre 10⁻¹² et 5% du poids total de la composition, de préférence entre 10⁻¹⁰ et 2% du poids total de la composition. Cette quantité correspond à la quantité efficace pour diminuer voire inhiber la dégradation des fibres de collagène induite en condition solaire naturelle sub-érythémale ou érythémale.
Cette quantité efficace peut être déterminée *in vitro* à partir de mesures de la diminution de la production de MMP-1 sur des cultures comprenant des kératinocytes et des fibroblastes soumis à une radiation solaire simulée ou un rayonnement UV caractérisé par un rapport UVA/UVB compris entre 10 et 17.

L'inhibiteur de facteurs cytosolubles kératinocytaires photoinduits selon l'invention pourra être associé à un autre inhibiteur de MMPs, ainsi qu'à des adjuvants cosmétiques classiques, tels que définis ci-après.

L'utilisation des inhibiteurs de facteurs cytosolubles kératinocytaires selon l'invention est ainsi adaptée à la préparation de compositions destinées à diminuer voire inhiber la production de la collagénase interstitielle MMP-1 au niveau du derme, *via* un mécanisme paracrine.

Selon un mode particulier de l'invention, l'inhibiteur de la production de facteurs cytosolubles kératinocytaires photoinduits tel que défini précédemment est associé à au moins un inhibiteur direct de la production de MMP-1.

Ces compositions seront utiles pour prévenir et/ou diminuer les signes du vieillissement cutané et la dégradation des fibres de collagène de sujets soumis à des conditions d'exposition solaire sub-érythémale ou érythémale.

Par inhibiteur 'direct' de la production, on entend, par opposition à l'inhibiteur objet de l'invention qualifié d'inhibiteur 'indirect', un inhibiteur agissant directement au niveau du derme sur la production et/ou de l'activation de MMP1.
On peut citer par exemple des inhibiteurs de la production de MMPs d'origine naturelle ou synthétique.
Par "origine naturelle", on entend un inhibiteur de métalloprotéinase à l'état pur ou en solution à différentes concentrations, obtenu par différents procédés d'extraction à partir d'un élément du règne animal et/ou végétal.
Par "origine synthétique", on entend l'inhibiteur de métalloprotéinase à l'état pur ou en solution à différentes concentrations, obtenu par synthèse chimique.

Comme exemples d'inhibiteurs de MMPs d'origine naturelle, on peut citer :
- un inhibiteur de métalloprotéinase choisi parmi les inhibiteurs de MMP-1, MMP-2, MMP-3 et MMP-9 ;
- un inhibiteur tissulaire des métalloprotéinases (TIMP) tel que les peptides connus dans l'art antérieur sous les appellations TIMP-1, TIMP-2, TIMP-3 et TIMP-4 (Woessner J. F., Faseb Journal, 1991) ;
- un extrait naturel contenant de l'acide Ursolique ou des caroténoïdes ou de la vitamine C ou les isoflavones comme la genistéine connue pour son activité inhibitrice de métalloprotéinase (US6130254) ;
- le lycopène ou une isoflavone et leurs dérivés. L'activité du lycopène sur les métalloprotéinases a été décrite dans la demande de brevet EP-A-1090628 ; parmi les extraits végétaux en contenant, on peut citer les extraits de soja (commercialisé par exemple par la société ICHIMARU PHARCOS sous la dénomination commerciale Flavostérone SB®), de trèfle rouge, de lin, de kakkon ou de sauge;
- un inhibiteur transcriptionnel de la métalloprotéinase MMP-1, tel que le rétinol ou ses dérivés, l'acide rétinoïque et ses dérivés ;
- des sapogénines, telles que la diosgénine, l'hécogénine, la smilagénine, la sarsapogénine, la tigogénine, la yamogénine et la yuccagénine ;
- la genisteine et la quercetine (US 5 637 703, US 5 665 367) ;
- des tétracyclines ;
- l'extrait de malt commercialisé par la société COLETICA sous la dénomination commerciale Collalift® ;
- les extraits de myrtille, de romarin, de sauge.

Comme exemples d'inhibiteurs de MMPs d'origine synthétique, on peut citer :
- l'adapalène ou encore des peptides analogues et/ou des dérivés du Batimastat ((BB 94 ) =[4-(N-hydroxyamino)-2R-isobutyl-3S-(thiophen-2-ylthiomethyl)-succinyl]-L-phenylalanine-N-methylamide), du Marimastat ((BB 2516)=[2S-[N4(R*), 2R*,3S]]-N4[2,2-dimethyl-1-[(methylamino)carbonyl]propyl]-N1,2-dihydroxy-3-(2-methylpropyl)butanediamide) vendus par la société British Biotech ;
- des dérivés de tétracyclines, tels que minocycline, roliteracycline, chlortetracycline, methacycline, oxytetracycline, doxycycline, demeclocycline et sels correspondants ;
- les oligopeptides et les lipopeptides, les lipoaminoacides.

Les utilisations décrites pécédemment sont donc adaptées à la préparation de compositions destinées à inhiber la dégradation des fibres de collagène, induite présence d'une exposition solaire naturelle sub-érythémale ou érythémale.

L'utilisation d'un inhibiteur de facteurs cytosolubles photoinduits selon l'invention sera avantageuse comme agent destiné à prévenir les signes cutanés liés à une dégradation du collagène photoinduite, cet agent étant efficace y compris en condition solaire sub-érythémale. En particulier, cette utilisation est destinée à prévenir l'amincissement de la peau et/ou la perte de tonicité de la peau et/ou la perte d'élasticité de la peau.

Ces compositions pourront notamment être utilisées sur les personnes à peaux et/ou cuirs chevelus sensibles, du fait de l'efficacité des inhibiteurs selon l'invention dans des conditions d'exposition solaire sub-érythémales.
Certaines personnes, dites à peaux sensibles ou réactives, ont en effet un niveau de neurosensibilité cutanée très bas et réagissent à des substances irritantes ou à des facteurs environnementaux dont le soleil de manière beaucoup plus intense que d'autres personnes ; elles se plaignent d'un inconfort cutané caractérisé par des sensations d'échauffements, de picotements, de fourmillements ou de démangeaisons non-allergiques.

Un autre objet de l'invention est une composition comprenant, dans un milieu physiologiquement acceptable, et selon une première alternative, une quantité efficace d'au moins un inhibiteur choisi parmi un inhibiteur de cyclooxygénases, un inhibiteur d'histones déacétylases, et leurs mélanges.
La composition peut être une composition cosmétique ou une composition dermatologique. De préférence, il s'agira d'une composition cosmétique.

Par 'milieu physiologiquement acceptable', on entend selon l'invention un milieu compatible avec la peau et/ou ses phanères (cils, ongles, cheveux) et/ou les muqueuses, et en particulier un milieu qui ne contiendra pas de solvant à effet irritant.

En particulier, la composition comprend une quantité efficace d'au moins un inhibiteur d'histones déacétylases, choisi parmi la trichostatin A, un acide gras à chaîne courte en C2 à C6 tel que l'acide propionique et/ou ses dérivés et/ou ses sels, l'acide butyrique et/ou des dérivés et/ou ses sels, et leurs mélanges.
Parmi les dérivés, on peut citer les esters, et en particulier ceux comprenant des radicaux sucres et hydroxydes, tels que le monoacétone glucose-3-propionate et le monoacétone glucose-3-butyrate.
Parmi les sels, on peut citer les sels organiques, tels que les ammoniums obtenus par réaction avec des amino-acides basiques, et les sels inorganiques obtenus par exemple par réaction avec les métaux alcalins (sodique ou potassique), les alcalino-terreux (Mg2+, Ca2+) et les métaux de transition (Fe, Mn, Co, Cu...).
De façon préférée, la composition comprend une quantité efficace d'acide butyrique et/ou ses dérivés et/ou ses sels, et en particulier le butyrate de sodium.
Et en particulier, elles se présente sous la forme d'une solution hydroalcoolique ou huileuse, d'une émulsion de type huile-dans-eau, eau-dans-huile ou d'une émulsion multiple, de gel aqueux ou anhydre, de microcapsules ou microparticules, ou de dispersions vésiculaires de type ionique et/ou non ionique.

Comme dérivés de l'acide butyrique utilisables dans les compositions de l'invention, on peut citer les composés de formule générale (I): 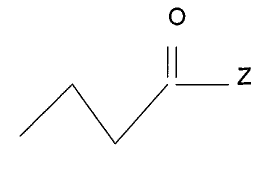 où Z peut être un groupe choisi parmi les groupes -OH, -OR', -NH2, -NHR', -NR'R" ; et R' et R" peuvent être des groupes aryle ou alkyle en C1-C8, linéaires ou ramifiés, saturés ou insaturés, éventuellement substitués par des groupes hydroxyles, alkoxy, acyloxy, amino, alkylamino, ou aryles, et/ou leurs sels.

Comme sels organiques d'acide butyrique ou de ses dérivés de formule (I) utilisables dans les compositions de l'invention, on peut citer les sels de tri-éthanolamine, mono-éthanolamine, di-éthanolamine, hexadécylamine et N,N,N',N'-tétrakis-(hydroxy-propyl-2) éthylène diamine, tris-hydroxyméthyl-aminométhane ou encore les sels d'acides carboxyliques comme les citrates, les acétates, les lactates, les fumarates, les gluconates, les oxalates pour les composés selon l'invention portant une fonction basique.

Comme sels inorganiques d'acide butyrique ou de ses dérivés de formule (I) utilisables dans les compositions de l'invention, on peut citer les sels d'acides minéraux comme les sels de sodium ou de potassium, les sels de zinc (Zn²⁺), de calcium (Ca²⁺), de cuivre (Cu²⁺), de fer (Fe²⁺, Fe³⁺), de strontium (Sr²⁺), de magnésium (Mg²⁺) et de manganèse (Mn²⁺) ; ou encore les hydroxydes, les carbonates ou hydrogénocarbonates, les chlorures, les sulfates, les phosphates ou hydrogénophosphates pour les composés selon l'invention portant une fonction basique.

De préférence, les compositions de l'invention comprendront tout sel inorganique d'acide butyrique à l'exception du butyrate de calcium. Et en particulier, elles contiendront du butyrate de sodium.

L'invention porte également sur une composition cosmétique ou dermatologique comprenant, dans un milieu physiologiquement acceptable, et selon une seconde alternative, au moins une quantité efficace d'un inhibiteur de facteurs cytosolubles kératinocytaires photoinduits tel que défini précédemment associé à au moins un inhibiteur direct de la production de MMP-1 actif au niveau du derme.

Dans le cadre de cette seconde alternative :
- l'inhibiteur de facteurs cytosolubles kératinocytaires photoinduits selon l'invention est préférentiellement choisi parmi un inhibiteur de la production de cytokines inflammatoires, un inhibiteur des cyclooxygénases et un inhibiteur des histones déacétylases, tel que l'acide alipoïque, la trichostatin A, l'acide propionique et/ou ses dérivés et/ou ses sels en particulier le propionate de sodium, et l'acide butyrique et/ou ses dérivés et/ou ses sels, en particulier le butyrate de sodium ;
- et l'inhibiteur direct de la production de MMP-1 actif au niveau du derme peut être choisi parmi TIMP-1 ; le lycopène ou une isoflavone et leurs dérivés ; le rétinol ou ses dérivés, l'acide rétinoïque et ses dérivés ; les extraits de sauge, myrtille, romarin ; l'adapalène ou encore des peptides analogues et/ou des dérivés du Batimastat ((BB 94 ) =[4-(N-hydroxyamino)-2R-isobutyl-3S-(thiophen-2-ylthiomethyl)-succinyl]-L-phenylalanine-N-methylamide), du Marimastat ((BB 2516)=[2S-[N4(R*), 2R*,3S]]-N4[2,2-dimethyl-1-[(methylamino)carbonyl]propyl]-N1,2-dihydroxy-3-(2-methylpropyl)butanediamide) ; des sapogénines, telles que la diosgénine, l'hécogénine, la smilagénine, la sarsapogénine, la tigogénine, la yamogénine et la yuccagénine ; la genisteine et la quercetine ; des tétracyclines et dérivés, tels que minocycline, roliteracycline, chlortetracycline, methacycline, oxytetracycline, doxycycline, demeclocycline et sels correspondants ; les oligopeptides et les lipopeptides, les lipoaminoacides.

Selon l'une ou l'autre de ces alternatives, ledit inhibiteur de la production de facteurs cytosolubles kératinocytaires photoinduits est généralement dans la composition en une quantité comprise entre 10⁻¹² et 5% du poids total de la composition, de préférence entre 10⁻¹⁰ et 2% du poids total de la composition. Cette quantité correspond à la quantité efficace pour diminuer voire inhiber la dégradation des fibres de collagène induite en condition solaire naturelle sub-érythémale ou érythémale. Elle peut être déterminée *in vitro* comme décrit précédemment.

La composition peut comprendre en outre de façon avantageuse un agent choisi parmi un agent stimulant la synthèse des macromolécules du derme ou empêchant leur dégradation, en particulier un agent stimulant la synthèse de collagène ou empêchant sa dégradation, un agent favorisant la pousse et/ou limitant la chute des cheveux, un agent anti-oxydant, un filtre UV, et leurs mélanges.

Parmi les 'agents stimulant les macromolécules du derme ou empêchant leur dégradation', on peut citer ceux qui agissent :
- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes.
- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3®; et l'extrait de Saccharomyces cerevisiae disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition des sérine protéases telles que l'élastase leucocytaire ou la cathepsine G. On peut citer: l'extrait peptidique de graines de légumineuse (Pisum sativum) commercialisé par la société LSN sous la dénomination commerciale Parelastyl® ; les héparinoïdes ; et les pseudodipeptides tels que l'acide {2-[acétyl-(3-trifluorométhyl-phényl)-amino]-3-méthyl-butyrylamino} acétique.

Comme 'agent favorisant la pousse et/ou limitant la chute des cheveux', on peut citer l'aminexil, le 6-0-[(9Z,12Z)-octadéca-9,12-diènoyl]hexapyranose, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les hydroxy-acides, les benzophénones et l'hydantoïne, l'acide rétinoïque ; les vitamines comme la vitamine D, les analogues de la vitamine B12 et le panthoténol ; les céramides en particulier les composés de type 2-amino-alcane-1,3-diol et leurs dérivés, en particulier le 2-N-(2-hydroxy-hexadécanoyl)-amino-octadécane-1,3-diol et le 2-oléoylamino-octadécane-1,3-diol ; les triterpènes comme l'acide ursolique et les composés décrits dans US 5529769, US 5468888, US 5631282 ; les agents antiprurigineux comme la thénaldine, la triméprazine ou la cyproheptadine ; les antiparasitaires, en particulier le métronidazole, le crotamiton ou les pyréthrinoïdes ; les antifongiques, en particulier l'octopirox et les composés appartenant à la classe des imidazoles tels que l'éconazole, le kétoconazole ou le miconazole ou leurs sels, les esters d'acide nicotinique, dont notamment le nicotinate de tocophérol, le nicotinate de benzyle et les nicotinates d'alkyles en C₁-C₆ comme les nicotinates de méthyle ou d'hexyle ; les agents antagonistes de calcium, comme la cinnarizine, le diltiazem, la nimodipine, vérapamil, l'alvérine et la nifédipine ; les hormones telles que l'estriol ou ses analogues, la thyroxine et ses sels, la progestérone ; les agents antiandrogènes tels que l'oxendolone, la spironolactone, le diéthylstilbestrol et la flutamide ; leurs mélanges.

Parmi les 'agents antioxydants ou antiradicalaires', on peut citer par exemple la vitamine E et ses dérivés tels que l'acétate de tocophéryle ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

La concentration de ces agents additionnels dans la composition selon l'invention peut varier de 0,0001 à 20% en poids et est de préférence comprise entre 0,001 et 5% en poids, par rapport au poids total de la composition.

Par 'filtres UV', on entend les agents photoprotecteurs inorganiques actifs dans l'UVA et/ou l'UVB (absorbeurs), hydrosolubles ou liposolubles ou bien insolubles dans les solvants cosmétiques couramment utilisés, ou agents photoprotecteurs organiques.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Les agents photoprotecteurs organiques plus particulièrement préférés sont choisis parmi les composés suivants : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyle)-s-triazine, Anisotriazine, Ethylhexyl triazone, Diethylhexyl Butamido Triazone, Methylène bis-Benzotriazolyl Tetramethylbutylphénol, Drometrizole Trisiloxane, 1,1-dicarboxy (2,2'-diméthyl-propyl)-4,4-diphénylbutadiène, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

Idéalement, l'inhibiteur de la production de facteurs cytosolubles kératinocytaires selon l'invention et les autres agents pourront être associés dans des préparations cosmétiques de soin des zones exposées au soleil (scalp, corps, visage, lèvres) et, de façon générale, dans toutes les préparations cosmétiques dites " anti-vieillissement cutané et "antichute de cheveux" qui ont pour objectif de ralentir la déstructuration chronobiologique des tissus de soutien et de l'architecture des éléments matriciels cutanés et capillaires.

Une composition préférée de l'invention est une composition à usage cosmétique.

Cette composition peut se présenter sous toutes formes galéniques connues adaptées au mode d'utilisation, en particulier adaptée à une application topique sur la peau et/ou le cuir chevelu.

Selon un mode particulier de l'invention, la composition selon l'invention est destinée à une administration orale, en particulier en 'cosmétique orale' : elle peut se présenter notamment sous forme de capsules, de gélules, dragées, de granulés, de pâte à mâcher, de gels, de sirops buvables,de comprimés ou de toute autre forme connue de l'homme du métier. En particulier, le(s) actif(s) selon l'invention peuvent être incorporés dans toutes formes de compléments alimentaires ou d'aliments enrichis, par exemple des barres alimentaires, ou des poudres compactées ou non. Les poudres peuvent être diluables à l'eau, dans du soda, des produits laitiers ou dérivés du soja, ou être incorporées dans des barres alimentaires.
Les actifs peuvent être formulés avec les excipients et composants usuels pour de tels compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.
Les agents de formulation et excipients pour composition orale, et notamment pour compléments alimentaires sont connus dans ce domaine et ne font pas ici l'objet d'une description détaillée.
Selon un autre mode particulier de l'invention, la composition selon l'invention est destinée à une application topique sur la peau : elle peut avoir la forme d'une solution ou suspension aqueuse, alcoolique ou hydro-alcoolique ou d'une suspension huileuse, d'une dispersion, d'une émulsion obtenue par dispersion d'une phase grasse dans une phase aqueuse (H/E) ou inversement (E/H) ou d'une émulsion multiple, d'un gel aqueux, hydro-alcoolique ou huileux, de microcapsules ou microparticules, de dispersions vésiculaires de type ionique et/ou non ionique.
En particulier, la composition pour application topique pourra être sous la forme d'une solution aqueuse, hydroalcoolique ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H) ou multiples, d'une poudre libre ou compactée à utiliser telle quelle ou à incorporer dans un milieu physiologiquement acceptable, ou de suspensions ou émulsions de consistance molle du type crème ou gel aqueux ou anhydres, ou encore de microcapsules ou microparticules, ou de dispersions vésiculaires de type ionique et/ou non ionique. Elle peut ainsi se présenter sous forme de crème, de poudre, de tampon imbibé, de solution, d'émulsion ou de dispersion vésiculaire, de lotion, de gel, de spray, de suspension, de shampooing, d'aérosol ou de mousse. Elle peut également consister en des préparations solides constituant des savons ou des pains de nettoyage.
Ces compositions sont préparées selon les méthodes usuelles.

De préférence, il s'agira d'une composition de jour ou d'une composition de protection solaire à effet protecteur contre la dégradation des fibres du collagène induite par une exposition solaire, même sub-érythémale.

La composition selon l'invention peut encore être destinée à application topique sur le cuir chevelu : elle peut se présenter sous forme d'une lotion de soin capillaire, de gel, d'un shampooing ou d'un après-shampooing capillaire, d'un savon liquide ou solide de nettoyage, d'un produit de mise en forme de la coiffure (laque, produit pour mise en pli, gel coiffant), d'un masque traitant, ou d'une composition de permanente ou de défrisage, de coloration ou décoloration des cheveux.

Les quantités des différents constituants du milieu physiologique de la composition selon l'invention sont ceux généralement utilisées dans les domaines considérés. En outre, ces compositions sont préparées selon les méthodes usuelles.

Lorsque la composition est une émulsion, la proportion de la phase grasse peut aller de 2 % à 80 % en poids, et de préférence de 5 % à 50 % en poids par rapport au poids total de la composition.
La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique. En plus des huiles, la phase grasse peut contenir des cires, des gommes, des polymères lipophiles, des produits "pâteux" ou visqueux contenant des parties solides et des parties liquides.
La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Les émulsionnants et co-émulsionnants utilisés pour l'obtention d'une composition sous forme d'émulsion sont ceux généralement utilisés dans les domaines cosmétique et pharmaceutique. Leur nature est, en outre, fonction du sens de l'émulsion. En pratique, l'émulsionnant et éventuellement le co-émulsionnant sont présents, dans la composition, en une proportion allant de 0,1 % à 30 % en poids, de préférence de 0,5 à 20 % en poids et mieux de 1 à 8%. L'émulsion peut, en outre, contenir des vésicules lipidiques et notamment des liposomes.
Comme émulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, alcool ou acide gras), les huiles d'origine animale (perhydrosqualène), les huiles de synthèse (ester d'acide gras, huile de Purcellin), les huiles siliconées (polydiméthylsiloxane linéaire ou cyclique, phényltriméthicone) et les huiles fluorées (perfluoropolyéthers). Comme cires, on peut citer les cires siliconées, les cires d'abeille, de carnauba ou paraffine, de polyéthylène.

De façon c onnue, la composition utilisée selon l'invention peut contenir également les adjuvants habituels dans les domaines concernés, tels que les conservateurs, les antioxydants, les solvants, les épaississants ou gélifiants de phase aqueuse ou de phase huileuse, les parfums, les charges, les pigments, les absorbeurs d'odeur et les matières colorantes, les électrolytes, les neutralisants, les actifs cosmétiques et pharmaceutiques et leurs mélanges.
Les quantités de ces différents adjuvants sont celles classiquement utilisées dans le domaine considéré, et par exemple de 0,01 à 50% du poids total de la composition, et mieux de 0,1 à 20% et par exemple de 0,1 à 10 %. Ces adjuvants, selon leur nature, peuvent être introduits dans la phase grasse, dans la phase aqueuse ou dans les vésicules lipidiques.

Bien entendu l'homme du métier veillera à choisir les éventuels additifs complémentaires et/ou leur quantité de telle manière que les propriétés avantageuses de la composition selon l'invention, à savoir l'inhibition de l'expression des collagénases au niveau du derme, ne soient pas ou substantiellement pas, altérées par l'adjonction envisagée.

L'invention porte également sur un procédé cosmétique destiné à prévenir et/ou diminuer et/ou traiter les signes cutanés du photo-vieillissement et/ou les atteintes liées à la ménopause, comprenant l'administration orale ou l'application topique sur la peau d'une composition cosmétique telle que définie ci-dessus.
En particulier, le procédé vise à prévenir et/ou traiter toute modification de l'aspect extérieur de la peau due au vieillissement photoinduit, comme les rides et ridules, la peau flétrie, la peau molle, la peau amincie, le manque d'élasticité et/ou de tonus de la peau.

Un autre objet de l'invention est un procédé cosmétique destiné à induire et/ou stimuler la croissance des cheveux et/ou freiner leur chute, caractérisé par le fait qu'il consiste à administrer oralement ou appliquer de façon topique sur le cuir chevelu, une composition cosmétique telle que définie précédemment, à laisser celle-ci en contact avec le cuir chevelu, et éventuellement à rincer le cuir chevelu.

De préférence, l'application se fera quotidiennement le matin (avant toute exposition à un rayonnement solaire naturel), notamment sur les parties du corps exposées habituellement au soleil comme le visage, les oreilles, le cou, les avant-bras, les mains et le cuir chevelu.
Ces applications peuvent être renouvelées pendant un ou plusieurs mois suivant les individus.

L'invention sera décrite plus en détail par les exemples ci-dessous, non limitatifs.

La figure 1 représente l'effet d'une exposition RSS pendant 24h sur la production de MMP-1 d'une peau humaine reconstruite *in vitro,* par mesure ELISA.

La figure 2 illustre le rôle de l'épiderme dans la production de MMP-1 d'une peau reconstruite *in vitro.* Les peaux humaines reconstruites ont été exposées aux RSS ou UV-B et la production de MMP-1 est évaluée par ELISA 24 h après. Alternativement, l'épiderme de la peau reconstruite a été enlevé immédiatement après exposition UV (barres noires).

La figure 3 représente les effets d'une exposition RSS sur la production de MMP-1 dans un épidémie reconstruit *EPISKIN™*. Le contenu en MMP-1 est évalué par ELISA 24h après dans l'épiderme (A), dans le gel de collagène acellulaire (B) et dans le milieu de culture (C).

La figure 4 est un histogramme représentant l'effet d'une exposition RSS sur la production de MMP-1 dans les fibroblastes du derme humain normal.

La figure 5 illustre la production de MMP-1 par les kératinocytes de l'épiderme humain normal. Les cellules cultivées sur plastique sont exposées à RSS ou traitées avec TPA (20ng/ml). Le contenu de MMP-1 est évalué par ELISA 24h après traitement dans le surnageant de culture cellulaire.

La figure 6 est un histogramme représentant l'effet du milieu conditionné de kératinocytes exposés à RSS sur la production de MMP-1 dans les fibroblastes de derme humain normal. Le milieu conditionné est collecté 24h après irradiation et transféré sur des fibroblastes de derme humain normal. La production de MMP-1 est évaluée dans le milieu de culture des fibroblastes du derme par ELISA, 24h après le transfert.

### EXEMPLES

### Exemple 1 : Mise en évidence du rôle de facteurs cytosolubles kératinocytaires dans la production de MMP-1 par les fibroblastes, dans des conditions d'exposition solaire naturelle simulée (RSS)

### - Matériels et méthodes

### Cultures cellulaires

Les kératinocytes humains sont isolés de peau humaine normale (chirurgie mammaire) et cultivés sur une couche nourricière de fibroblastes de souris 3T3 traités à la mitomycine.

Les fibroblastes de derme humain sont isolés à partir d'explants mammaires amplifiés de peau et cultivés sur milieu Dulbecco Eagle modifié (DMEM) contenant 10% FCS, supplémenté en glutamine (2mM), pyruvate de sodium (1 mM) et acides aminés non essentiels.
Toutes les cultures de cellules sont maintenues à 37°C dans 5% CO₂.

Les peaux reconstruites sont composées d'un équivalent de derme vivant et d'un épiderme différencié, préparés comme décrit dans Asselineau et al. (1985), Exp. Cell. Res. 159, 536-539.

Un modèle d'épiderme humain reconstruit, EPISKIN™ (EPISKIN SNC, France) a également été utilisé. Des kératinocytes mammaires humains ont été ensemencés sur un substrat de collagène sans cellule et mis à pousser jusqu'à différenciation épidermique complète.

Une peau humaine *'ex vivo'* est obtenue à partir d'une chirurgie du sein. Des biopsies de 6mm de peau humaine ont été réalisées et maintenues pendant 24h en culture à l'interface air-liquide sur une grille (*ex vivo*) dans les mêmes conditions que pour une peau reconstruite.

### Radiation solaire simulée (RSS)

Une radiation solaire simulée (RSS) est obtenue en utilisant une lampe au xénon de 1000 watt (Oriel corp. CT) équipée de filtres Schoot UG 5/2mm et WG 320/1.5mm. Cette source au xénon filtrée fournit un spectre UV solaire simulé (290-400nm). La sortie spectrale délivrée est de 9% en UV-B, correspondant à une condition solaire réelle au zénith. Un tube Philips TL 20W/12 équipé d'un filtre Kodacel pour éliminer les longueurs d'ondes en dessous de 290nm est utilisé comme source d'UV-B. Le spectre de longueur d'onde est vérifié attentivement avec un spectroradiomètre Macam SR3010.

Juste avant l'irradiation, le milieu de culture des cellules sub-confluentes (80-90% de confluence) est remplacé par un tampon phosphate PBS pour éviter la génération de produits phototoxiques par le milieu. Dans ces conditions, aucune perte de viabilité des cellules n'est observée aux doses UV utilisées.

Après l'irradiation, le tampon PBS est remplacé par le milieu des kératinocytes ou le milieu des fibroblastes sans sérum ni facteurs de croissance connus pour interférer avec la détection ou la production de MMP-1.

### Analyse biochimique de la production de MMP-1

### - ELISA (détection)

Le contenu de MMP-1 est mesuré par ELISA (Biotrak kit RPN 2610, Amersham Biotech, France) selon les instructions du fournisseur.

### - Western blot (analyse quantitative)

La production de MMP-1 est également évaluée par la méthode standard de Western blot dans des conditions réductrices sur gel de 10% polyacrylamide, transféré sur membranes Hybond N (Amersham Biotech).
Les membranes sont incubées avec un anticorps monoclonal de souris anti-MMP-1 humaine pendant 1 h à température ambiante puis lavées.
Les membranes sont ensuite incubées avec un anticorps anti-souris couplé à la péroxydase pendant 45 minutes à température ambiante, lavées, et révélées en présence d'un système chémoluminescent ECL (Amersham biotech).

### - Zymogrammes (activité enzymatique)

Des gels de zymogrammes de NOVEX ont été utilisés pour étudier les métalloprotéinases à travers la détection de leur activité protéolytique.

Les échantillons ont été analysés en SDS-PAGE sans chauffage ni réduction, contenant de la caséine bleue pour détecter la collagénase. Après électrophorèse sur gel, les gels sont lavés 2 fois dans un tampon renaturant pour zymogramme (Biorad). Les protéines caséinolytiques sont identifiées par des bandes claires sur fond noir après marquage des protéines au bleu de coomassie (Pierce, France).

### - Résultats

### Détermination d'une dose efficace biologique sur peau ex-vivo

Dans la peau humaine *in vivo,* une exposition au soleil induit la formation dans l'épiderme de cellules 'coups de soleil', identifiées par une forme ronde, une perte de connection avec les kératinocytes, une localisation suprabasale, et un cytoplasme éosinophile autour d'un noyau dense et contracté.
Sur une peau humaine *ex vivo*, des analyses histologiques d'échantillons pris 24h après une exposition à une radiation solaire simulée révèlent l'apparition de cellules 'coups de soleil' dans la couche suprabasale de l'épiderme. Les expériences de dose-réponse ont déterminé une dose biologique efficace (DBE) autour de 10J/cm2: cette dose correspond à la dose minimale RSS induisant la formation de cellules 'coups de soleil'.

La production de MMP-1 dans le milieu de culture après irradiation UV a donc été évaluée en utilisant des doses de radiation solaire simulée (RSS) autour de la dose biologique efficace (DBE). Les résultats ELISA et Western blot montrent que l'exposition RSS induit une augmentation du niveau de protéine MMP-1 sécrétée, de façon dose dépendante. L'analyse du zymogramme révèle que cet effet est associé à une augmentation de l'activité protéolytique de MMP-1 dans le milieu de culture.

### Production de MMP-1 à partir d'une peau reconstruite in vitro soumise à RSS

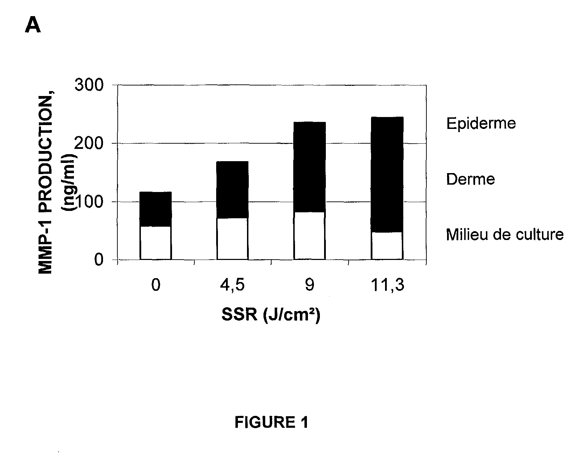

Comme pour la peau ex *vivo,* la formation de cellules 'coups de soleil' est détectée sur une peau reconstruite *in vitro* à une dose RSS de 9J/cm2 et est également dose dépendante.
En réponse à une exposition de type RSS, une augmentation dose-dépendante du contenu en protéine MMP-1 peut être détectée par ELISA dans le milieu de culture et le derme équivalent mais pas dans l'épiderme (Figure 1).
Les Western blots confirment ce résultat ; de plus, l'analyse Western blot révèle également que la protéine MMP-1 est trouvée dans sa forme inactive proMMP (zymogène) et sa forme active dans l'équivalent de derme, mais principalement dans sa forme inactive dans le milieu de culture.

L'analyse du zymogramme confirme une augmentation de l'activité protéolytique après exposition RSS dans le derme et dans une moindre mesure dans le milieu de culture mais pas dans l'épiderme.

Ces résultats indiquent que la localisation de MMP-1 induite par une exposition RSS est préférentiellement dans le derme.
On sait par ailleurs que :
- la radiation UV-A seule est capable d'induire la production de MMP-1 dans une peau humaine reconstruite *in vitro, via* un effet direct des UV-A sur les fibroblastes du derme (Bernerd F et al. 1998, Cell Death Differ, 5, 792-802) ;
- une radiation UV-B, dont la cible est préférentiellement l'épiderme du fait d'une plus faible pénétration, utilisée sur une peau reconstruite *in vitro,* à une dose biologique efficace, est é galement capable d'induire une production de MMP-1 détectée dans le milieu de culture d'échantillons irradiés.

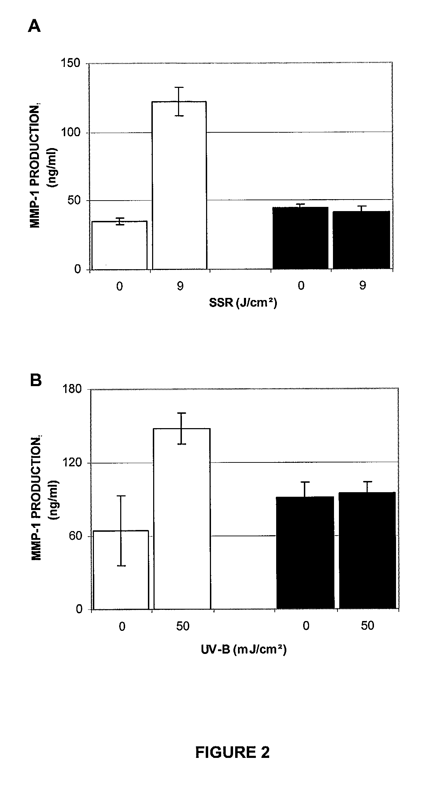

Cependant, l'enlèvement de la couche d'épiderme par peeling, juste après l'exposition de type RSS ou source UV-B abroge la production de MMP-1, suggérant une participation essentielle de l'épiderme dans cette induction (Figure 2).

Les expériences suivantes ont été réalisées pour déterminer le rôle direct et/ou indirect de l'épiderme dans cette induction.

### Production de MMP-1 à partir d'un épiderme reconstruit in vitro soumis à RSS

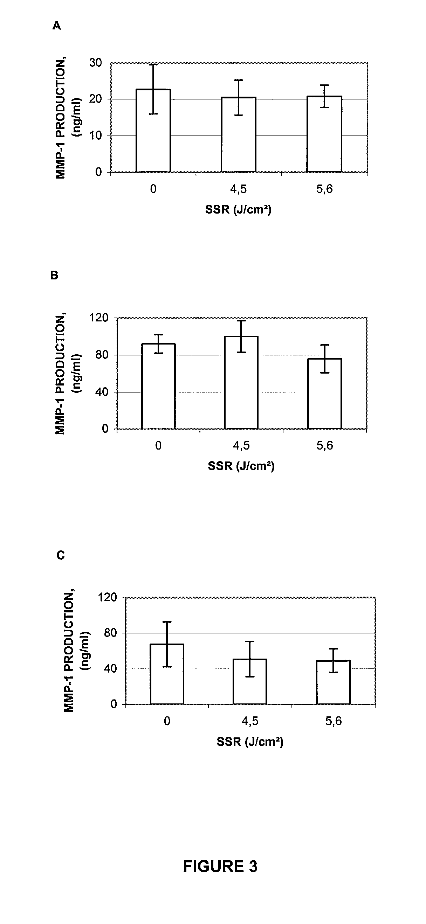

Une exposition de type RSS du modèle EPISKIN™ à une dose capable d'induire la formation de cellules 'coups de soleil' ne conduit pas à une induction de la production de MMP-1 dans le feuillet épidermal, ni dans le gel de collagène acellulaire ni dans le milieu de culture EPISKIN™ (Figure 3).
Pris ensemble avec les résultats précédents, ces résultats suggèrent que les fibroblastes du derme jouent un rôle essentiel dans la production de MMP-1 après une exposition de type RSS et que l'épiderme, bien que non responsable d'une production directe de MMP-1, participerait indirectement à l'induction de MMP-1 après une exposition de type RSS.

### Production de MMP1 à partir de cultures de kératinocytes et fibroblastes séparées, soumises à RSS

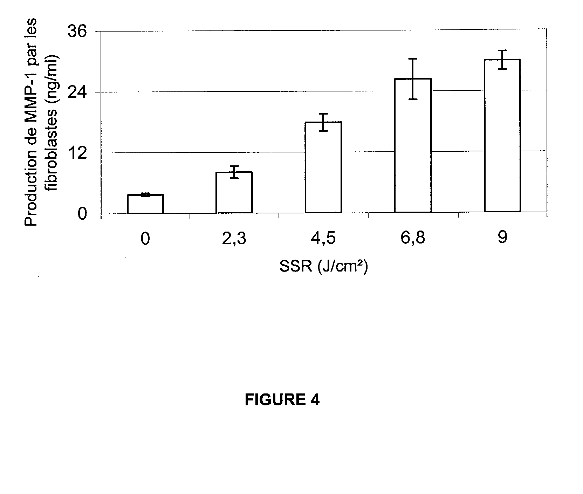

Des précédents travaux ont bien documenté la capacité des UV-A et UV-B utilisés séparément à stimuler la production de MMP-1 dans les cultures de fibroblastes. En accord avec ces études, on a pu montrer que 24h après une exposition de type RSS, les fibroblastes du derme humain normal en croissance sur du plastique présentaient une augmentation dose dépendante de production de MMP-1 dans le surnageant de culture cellulaire quantifié par ELISA ou détecté par western blot et analyse zymographique (Figure 4).

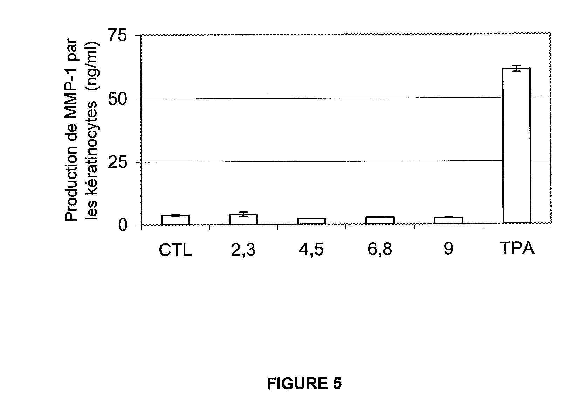

Pour déterminer si l'irradiation de type RSS pouvait aussi moduler la production de MMP-1 par des kératinocytes en croissance sur du plastique, les cellules ont été exposées à une radiation RSS. Les contenus extracellulaires de MMP-1 ont été quantifiés 24h après la radiation UV. Par contraste avec les fibroblastes, l'exposition de type RSS sur les kératinocytes ne conduit pas à une augmentation de production de MMP-1. Cependant, la stimulation des kératinocytes par le TPA (Trans Plasminogen activator, un activateur de protéine kinase C), utilisé comme contrôle positif, conduit à une accumulation de production de MMP-1 (Figure 5).
Ces résultats indiquent que les kératinocytes sont capables de produire MMP-1 en réponse à un traitement approprié mais pas en réponse à une exposition RSS.

### Action de l'épiderme (kératinocytes) sur la production de MMP-1 par les fibroblastes du derme via un mécanisme paracrine

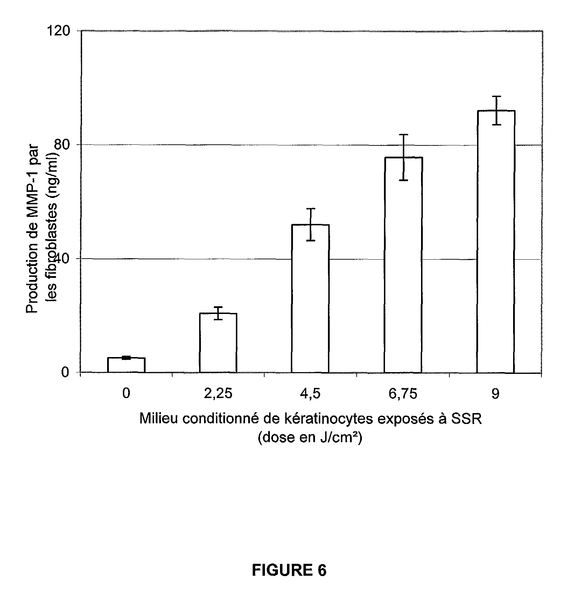

Pour tester l'implication d'un mécanisme paracrine, sachant que la production de MMP-1 par les fibroblastes est régulée par des modulateurs positifs comme les cytokines, des kératinocytes humains normaux ont été exposés à une radiation de type RSS et les milieux de culture ont été collectés 24h après.
Ensuite, les fibroblastes de derme humain sont traités avec le milieu conditionné obtenu des kératinocytes exposés à RSS ; ce milieu conditionné contient des facteurs cytosolubles kératinocytaires et, notamment des facteurs cytosolubles photoinduits.
Le contenu MMP-1 est ensuite évalué dans le milieu de culture des fibroblastes 24h après.
Les résultats montrent que le milieu conditionné des kératinocytes exposés à RSS est capable de réguler positivement de façon dose dépendante la production de MMP-1 par les fibroblastes humains, détectée par ELISA (Figure 6)
Dans les conditions expérimentales, la production de MMP-1 par les fibroblastes du derme, induite par le milieu conditionné, est nettement supérieure à la production de MMP-1 directement induite par la radiation de type RSS (18 fois contre 8 fois).

Ces données indiquent q ue le développement de dommages cellulaires au niveau du derme, induit par un rayonnement solaire, serait dû à des effets directs spécifiques au niveau du derme, principalement induits par des longueurs d'ondes UV-A pénétrantes. Cependant, une participation significative du compartiment épidermique est impliquée dans la libération de facteurs solubles kératinocytaires, qui serait grandement influencée par les longueurs d'ondes UV-B, moins pénétrantes.

### Exemple 2 : Sélection d'inhibiteurs de la production de facteurs cytosolubles kératinocvtaires photoinduits

Des kératinocytes humains normaux cultivés en monocouche sur plastique ont été soumises à un rayonnement UV-B (10mJ/cm²). L'expérience peut également être réalisée en présence d'une RSS telle que définie à l'exemple 1.
Un tube Philips TL 20W/12 équipé d'un filtre Kodacel pour éliminer les longueurs d'ondes en dessous de 290nm est utilisé comme source d'UV-B.
Juste avant l'irradiation, le milieu de culture des cellules sub-confluentes (80-90% de confluence) est remplacé par un tampon phosphate PBS pour éviter la génération de produits phototoxiques par le milieu. Dans ces conditions, aucune perte de viabilité des cellules n'est observée aux doses UV utilisées.
Après irradiation, les cellules ont été mises au contact d'un milieu de culture MEM sans sérum ni facteur de croissance connus pour interférer avec la détection ou la production de MMP-1 contenant ou non du butyrate de sodium BuNa (3mM).

Le milieu conditionné des cellules kératinocytaires (irradiées ou non en absence ou en présence de butyrate de sodium) a été prélevé 24h après irradiation et a été mis au contact de fibroblastes humains normaux.
La production de MMP-1 détectée 24h plus tard dans le surnageant de culture des fibroblastes a été quantifée grâce au kit de détection « Biotrack MMP-1 human ELISA system » commercialisé par Ammersham, selon les indications du fournisseur.
La lecture des résultats est réalisée à l'aide d'un lecteur de microplaques LabSystems équipé d'un filtre de 450nm.
La quantité de MMP-1 est calculée par rapport à une gamme étalon réalisée avec un standard fourni avec le kit, et est exprimée en ng/ml.

Les résultats sont les suivants :

| **Conditons expérimentales** | **Production de MMP-1 (ng/ml)** |
|---|---|
| Milieu conditionné contrôle kératinocytes non irradiés | 13.80 ± 1.01 |
| Milieu conditionné kératinocytes non irradiés, traités au butyrate de sodium (NaBu) | 4.84 ± 0.36 |
| Milieu conditionné kératinocytes irradiés | 40.70 ± 0.64 |
| Milieu conditionné kératinocytes irradiés traité au butyrate de sodium (NaBu) | 16.97 ± 3.7 |

En condition contrôle (non irradiée), la production de MMP-1 correspond au taux basal de facteurs cytosolubles kératinocytaires présents dans nos conditions de culture.
La présence du butyrate de sodium inhibe cette production de MMP-1 'basale' d'un facteur 3.

En condition UV-B, on obtient une surproduction de MMP-1, d'un facteur 3 par rapport à la condition contrôle (non irradiée), due à la présence de facteurs cytosolubles kératinocytaires photoinduits.
Le butyrate de sodium, additionné au milieu conditionné de k ératinocytes irradiés, est capable d'empêcher cet effet stimulateur sur la production de MMP-1, en inhibant la production de facteurs cytosolubles kératinocytaires photoinduits présents dans le milieu.

Ces résultats montrent donc que le butyrate de sodium est un inhibiteur 'indirect' de choix pour des applications cosmétiques visant à prévenir et/ou traiter la dégradation du collagène au niveau du derme liée à une exposition solaire.

### Exemple 3 : Formulations

### Composition 1 : Crème de soin du visage

On prépare l'émulsion huile-dans-eau suivante de façon classique (% en poids) :
- Butyrate de sodium 1%
- Lycopène (sous forme de Lycomato® à 10% d e Lycopène dans une Oléorésine de tomate commercialisée par Lycored®) 0,001 %
- Stearate de glycérol 2 %
- Polysorbate 60 (Tween 60® vendu par la société ICI) 1 %
- Acide stéarique 1,4 %
- Triéthanolamine 0,7 %
- Carbomer 0,4 %
- Fraction liquide du beurre de karité 12 %
- Perhydrosqualène 12 %
- Antioxydant qs
- Parfum qs
- Conservateur qs
- Eau qsp 100 %

### Composition 2 : Gel pour le soin du visage

| | |
|---|---|
| Butyrate de sodium | 10⁻⁵ % |
| Hydroxypropylcellulose (Klucel H® vendu par la société Hercules) | 1,00 % |
| Antioxydant | 0,05 % |
| Isopropanol | 40,00 % |
| Conservateur | 0,30 % |
| Eau | qsp 100,00 % |

### Composition 3 : Lotion capillaire antichute

| | |
|---|---|
| - monoacétone glucose-3-butyrate | 1% |
| - lycopène (sous forme de Lycomato® à 10% de Lycopène dans une Oléorésine de tomate commercialisée par Lycored®) | 0.0001% |
| - propylène glycol | 30% |
| - éthanol | 55% |
| - eau | qsp 100% |

### Composition 4 : gelule gélatine végétale ou animale

| | |
|---|---|
| | mg/ gélule |
| - butyrate de sodium | 10 |
| - amidon | 198 |
| - stéarate de magnésium | 2,5 |

## Revendications

1. Utilisation, pour la préparation d'une composition cosmétique ou dermatologique destinée à prévenir et/ou lutter contre la dégradation des fibres de collagène induite par un rayonnement solaire **caractérisé par** un rapport UVA/UVB compris entre 10 et 17, d'une quantité efficace d'au moins un inhibiteur de la production de facteurs cytosolubles kératinocytaires photoinduits.

2. Utilisation selon la revendication 1, **caractérisée par le fait que** l'inhibiteur de la production de facteurs cytosolubles kératinocytaires photoinduits est susceptible d'être sélectionné selon le procédé comprenant les étapes suivantes:
a- on soumet des kératinocytes humains normaux cultivés en monocouche à une radiation solaire simulée ou un rayonnement UV, **caractérisés par** un rapport UVA/UVB compris entre 10 et 17 ;
b- on met les cellules kératinocytaires irradiées ou non au contact d'un milieu de culture contenant ou non le produit à tester ;
c- on met le milieu conditionné issu de b) au contact de fibroblastes humains normaux cultivés en monocouche ;
d- on mesure la production de collagénase interstitielle MMP-1 dans le surnageant de culture des fibroblastes issu de c), que l'on compare à un témoin constitué d'une culture de fibroblastes normaux humains mis en contact avec un milieu conditionné de cellules kératinocytaires non irradiées ; et
e- on sélectionne les produits pour lesquels la mesure de la production de collagénase interstitielle MMP-1 est inférieure d'au moins 10% à celle obtenue pour le témoin, préférentiellement inférieure d'au moins 20%, et encore plus préférentiellement inférieure d'au moins 30% à celle obtenue pour le témoin.

3. Utilisation selon l'une des revendications 1 ou 2, **caractérisée par le fait que** l'inhibiteur de la production de facteurs cytosolubles kératinocytaires photoinduits est choisi parmi un inhibiteur de la production de cytokines inflammatoires, un inhibiteur de cyclooxygénases, un inhibiteur d'histones déacétylases et leurs mélanges.

4. Utilisation, pour la préparation d'une composition cosmétique ou dermatologique destinée à prévenir et/ou lutter contre la dégradation photoinduite des fibres de collagène, d'une quantité efficace d'au moins un inhibiteur de facteurs cytosolubles kératinocytaires photoinduits choisi parmi un inhibiteur d'histones déacétylases, un inhibiteur de cyclooxygénases et leurs mélanges.

5. Utilisation selon l'une des revendications 3 ou 4, **caractérisée par le fait que** l'inhibiteur d'histones déacétylases est choisi parmi l'acide α-lipoïque, la trichostatin A, un acide gras à chaîne courte en C2 à C6 tel que l'acide propionique et/ou ses dérivés et/ou ses sels et l'acide butyrique et/ou ses dérivés et/ou ses sels, et leurs mélanges.

6. Utilisation selon la revendication 5, **caractérisée par le fait que** l'inhibiteur d'histones déacétylases est le butyrate de sodium.

7. Utilisation selon l'une des revendications 1 à 6, **caractérisée par le fait que** la composition comprenant une quantité efficace d'au moins un inhibiteur de la production de facteurs cytosolubles kératinocytaires photoinduits est destinée à diminuer voire inhiber la production de la collagénase interstitielle MMP-1 au niveau du derme, via un mécanisme paracrine.

8. Utilisation selon l'une des revendications 1 à 7, **caractérisée en ce que** l'inhibiteur de la production de facteurs cytosolubles kératinocytaires photoinduits est associé à au moins un inhibiteur direct de la production de MMP-1.

9. Utilisation selon l'une des revendications 1 à 8, **caractérisée par le fait que** la composition est destinée à prévenir et/ou lutter contre la dégradation des fibres de collagène, induite en présence d'une exposition solaire naturelle sub-érythémale ou érythémale.

10. Utilisation selon l'une des revendications 1 à 9, **caractérisée en ce que** l'inhibiteur de la production de facteurs cytosolubles kératinocytaires photoinduits ou la composition est destiné à prévenir l'amincissement de la peau, la perte de tonicité et/ou la perte d'élasticité de la peau.

11. Utilisation selon l'une des revendications 1 à 10, **caractérisée en ce que** la composition est adaptée à une administration par voie orale ou une application par voie topique.

12. Composition cosmétique pour application topique sur la peau et/ou le cuir chevelu comprenant, dans un milieu physiologiquement acceptable, une quantité efficace d'au moins d'un inhibiteur d'histones déacétylases choisi parmi l'acide butyrique, ses dérivés, ses sels, et leurs mélanges, ladite composition étant sous la forme d'une solution hydroalcoolique ou huileuse, d'une émulsion de type huile-dans-eau, eau-dans-huile ou d'une émulsion multiple, de gel aqueux ou anhydre, de microcapsules ou microparticules, ou de dispersions vésiculaires de type ionique et/ou non ionique.

13. Composition selon la revendication 12, **caractérisée en ce que** le sel d'acide butyrique est le butyrate de sodium.

14. Composition comprenant, dans un milieu p hysiologiquement a cceptable, a u moins une quantité efficace d'inhibiteur de la production de facteurs cytosolubles kératinocytaires photoinduits tel que défini dans l'une des revendications 2 à 6 associé à au moins un inhibiteur direct de la production de MMP-1.

15. Composition selon la revendication 14, **caractérisée en ce que** l'inhibiteur direct de la production de MMP-1 est choisi parmi TIMP-1 ; le lycopène ou une isoflavone et leurs dérivés ; le rétinol ou ses dérivés, l'acide rétinoïque et ses dérivés ; les extraits de sauge, myrtille, romarin ; l'adapalène ou encore des peptides analogues et/ou des dérivés du Batimastat ((BB 94 ) =[4-(N-hydroxyamino)-2R-isobutyl-3S-(thiophen-2-ylthiomethyl)-succinyl]-L-phenylalanine-N-methylamide), du Marimastat ((BB 2516)=[2S-[N4(R*), 2R*,3S]]-N4[2,2-dimethyl-1-[(methylamino)carbonyl]propyl]-N1,2-dihydroxy-3-(2-methylpropyl)butanediamide) ; des sapogénines, telles que la diosgénine, l'hécogénine, la smilagénine, la sarsapogénine, la tigogénine, la yamogénine et la yuccagénine ; la genisteine et la quercetine ; des tétracyclines et dérivés, tels que minocydine, roliteracycline, chlortetracycline, methacycline, oxytetracycline, doxycycline, demeclocycline et sels correspondants ; les oligopeptides et les lipopeptides, les lipoaminoacides.

16. Composition selon l'une des revendications 12 à 15, **caractérisée en ce que** l'inhibiteur de la production de facteurs cytosolubles kératinocyaires photoinduits est en une quantité comprise entre 10⁻¹² et 5% du poids total de la composition, de préférence entre 10⁻¹⁰ et 2% du poids total de la composition.

17. Composition selon l'une des revendications 12 à 16, **caractérisée en ce qu'**elle comprend en outre un agent choisi parmi un agent stimulant la synthèse des macromolécules du derme ou empêchant leur dégradation, en particulier un agent stimulant la synthèse de collagène ou empêchant sa dégradation, un agent favorisant la pousse et/ou limitant la chute des cheveux, un agent anti-oxydant, un filtre UV, et leurs mélanges.

18. Composition selon l'une des revendications 14 à 17, **caractérisée en ce qu'**elle est sous une forme adaptée à une administration orale.

19. Composition selon l'une des revendications 14 à 17, **caractérisée en ce qu'**elle est sous une forme adaptée à une application topique sur la peau et/ou le cuir chevelu.

20. Procédé cosmétique destiné à prévenir et/ou diminuer et/ou traiter les signes cutanés du vieillissement photo-induit et/ou les atteintes liées à la ménopause, **caractérisé par le fait que** l'on administre ou que l'on applique sur la peau une composition cosmétique selon l'une des revendications 12 à 19.

21. Procédé cosmétique destiné à induire et/ou stimuler la croissance des cheveux et/ou freiner leur chute, **caractérisé par le fait que** l'on administre ou que l'on applique sur le cuir chevelu une composition cosmétique selon l'une des revendications 12 à 19.
